# EUROPEAN PATENT SPECIFICATION

(11) **EP 1 313 489 B1**
(45) Date of publication and mention of the grant of the patent: **23.02.2005**
(21) Application number: 01980213.1
(22) Date of filing: 18.07.2001
(51) Int. Cl.: A61K 31/79

(54) **PHARMACEUTICAL COMPOSITIONS FOR THE TREATMENT OF MUCOSITIS, STOMATITIS AND BEHCET'S SYNDROME**
ARZNEIMITTEL ZUR BEHANDLUNG DER MUCOSITIS, STOMATITIS UND DES BEHCETSCHEN SYNDROMS
COMPOSITIONS PHARMACEUTIQUES POUR LE TRAITEMENT DE LA MUCOSITE, STOMATITE ET LE SYNDROME DE BEHCET

(30) Priority: 28.07.2000 IT MI001732
(43) Date of publication of application: 28.05.2003
(73) Proprietor: Sinclair Pharmaceuticals Limited, Godalming, Surrey GU7 2AB (GB)
(72) Inventor: MASTRODONATO, Marco, I-20121 Milano (IT)
(74) Representative: Minoja, Fabrizio, Dr.
(86) International application number: PCT/EP2001/008303
(87) International publication number: WO 2002/009637

(56) References cited:
- EP-A- 0 444 492
- GB-A- 2 092 442
- DATABASE BIOSIS [Online] BIOSCIENCES INFORMATION SERVICE, PHILADELPHIA, PA, US; T. HASEGAWA ET AL.: "Reparative effects of sodium alginate alloid G on radiation stomatitis" retrieved from BIOSIS Database accession no. PREV199089028433 XP002212190 & NIPPON ACTA RADIOLOGICA, vol. 49, no. 8, 1989, pages 1047-1051,

## Description

The present invention relates to pharmaceutical compositions containing hyaluronic acid, glycyrrhetinic acid and polyvinylpyrrolidone for the management of painful ulcerative and inflammatory conditions of moist surfaces including the mouth, oropharynx, oesophagus, vagina and rectum (including, but not limited to, mucositis, stomatitis, Behcet's syndrome).

### TECHNICAL BACKGROUND OF THE INVENTION

The terms mucositis and stomatitis are often used interchangeably but may include some general distinctions. Mucositis describes a toxic inflammatory reaction affecting the gastrointestinal tract, which may result from exposure to chemotherapeutic agents or ionising radiation. Mucositis typically manifests as an erythematous, burn-like lesion or as random, focal-to-diffuse, ulcerative lesions.

Stomatitis refers to an inflammatory reaction affecting the oral mucosa, with or without ulceration, that may be caused or intensified by pharmacological, particularly chemotherapeutic treatments, or by radiotherapy. Stomatitis can range from milt to severe; the patient with severe stomatitis is unable to take anything by mouth.

Many women get oral aphthous ulceration at specific times of the menstrual cycle and simultaneously get the same kind of ulcers in the genital tract, in particular the vulva and vagina. This is sometimes very severe and can cause retention of urine and require strong painkillers and sedatives. The most severe form is called Behcet's syndrome.

In the following description, the more general term mucositis will be employed also to indicate stomatitis proper. The study in mucositis exemplifies the action and supports the claim.

Erythematous mucositis may appear as early as three days after exposure to chemotherapy or radiotherapy, but more typically within five to seven days. Progression to ulcerative mucositis typically occurs within seven days after the start of chemotherapy and may sometimes reach such severity as to make it necessary to discontinue the pharmacological treatment. Mucositis may involve the mouth and oropharynx as well as the gastro-intestinal tract from the mouth to the anus. Here we confine the experience to mucositis affecting the easily accessible regions such as mouth, oropharynx, oesophagus and rectum.

As a high percentage (from 30 to 40%) of patients who receive chemotherapy will develop mucositis to varying degrees, there is a strong need for an effective, convenient treatment. To date, no effective treatments are in fact available and attempts have been made to solve the problem by use of analgesics, antiseptics and oral hygiene measures or palliation of symptoms.

Furthermore, the problem is not restricted to cancer patients, as mucositis frequently also occurs in HIV patients, particularly when associated with Kaposi's sarcoma, in patients affected with non-Hodgkin's lymphoma, in debilitated elderly patients and in patients receiving BRM treatments like interleukin-2, TNF, interferons, lymphokine-activated lymphocytes and the like.

GB-A-2092442 discloses water soluble pharmaceutical compositions comprising glycyrrhetinic acid or a derivative thereof useful for treating diseases of the oral cavity.

EP-A-0444492 discloses the use of hyalyronic acid and of its sodium salt for the topical application in the oral cavity.

### DISCLOSURE OF THE INVENTION

It has now been surprisingly found that the topical administration of a pharmaceutical formulation containing hyaluronic acid, glycyrrhetinic acid and polyvinylpyrrolidone provides an effective therapeutical and preventive treatment for mucositis and stomatitis of various origin and severity and, more generally, of the lesions of the oro-pharynx cavity and oesophagus, particularly those caused by dental devices and by radio- or chemotherapy.

Therefore the present invention, in a first aspect, provides pharmaceutical compositions comprising as active ingredients effective doses of hyaluronic acid, glycyrrhetinic acid and polyvinylpyrrolidone, in mixture with excipients and adjuvants to form a viscous and lubricating substance that remains adherent to the surface epithelium suitable for topical administration to epithelial surfaces such as, but not limited to, the oropharynx and oesophagus.

A further aspect of the invention concerns the use of hyaluronic acid, glycyrrhetinic acid and polyvinylpyrrolidone for the preparation of medicaments for the topical treatment of inflammatory states of epithelial surfaces such as, but not limited to the oral mucosa, particularly mucositis and stomatitis.

### DETAILED DISCLOSURE OF THE INVENTION

The compositions of the invention are in the form of a slightly viscous aqueous liquid (gel) which provides a film-forming and coating effect on the epithelial surfaces such as, but not limited to the oral mucosa.

Hyaluronic acid is present in weight percentages ranging from 0.01 to about 5%, preferably about 0.1%. Hyaluronic acid can also be in the form of the sodium salt, and preferably of biotechnological origin, with the molecular weight ranging from 1.6 and 2.2 10⁶ Da.

Polyvinylpyrrolidone or povidone (PVP), a suspending and binding agent widely used in pharmaceutical technique. It is present in the formulations of the invention in weith percentages ranging from 1 and 20% by weight, preferably between 5 and 10%.

Glycyrrhetinic acid can be present in weight percentages ranging from 0.01 to 3% by weight.

High molecular weight povidone is preferably used, for example povidones of K-30 to K-120, preferably povidone K-90, having an average molecular weight of about 1,000,000.

The compositions of the inventions can contain suitable excipients for topical administration such as:
- viscosity-increasing agents;
- surfactants;
- stabilising agents - preservatives;
- flavours, fragrances, sweetening agents;
- bioadhesives;
- co-solubilisers.

Examples of said excipients comprise cellulose derivatives, acrylic or methacrylic acids polymers or copolymers, ethylene or propylene glycols, polyethoxylated hydrogenated castor oil, EDTA, sodium benzoate, sodium or potassium sorbate, dextrins, sodium saccharin, aspartame and other excipients conventionally used in the formulation of collutories or liquid oral forms.

The compositions of the invention may further contain other active ingredients with complementary or anyway useful activity, such as antibacterials/ disinfectants, antifungals, analgesics, anti-inflammatories, emollients, local anæsthetics and the like. Suitable antimicrobials include quaternary ammonium salts such as benzalkonium chloride.

Finally, the compositions of the invention can be presented as single- or multi-dose forms, for example in sachets, vials, ampoules, bottles and the like.

Dosage will depend on a number of factors, such as severity, type and extension of the disease to treat: in principle, however, a wash or gargle with 10-50 ml of solution, optionally diluted in water, for a time of about up to two or three minutes three times or more daily, preferably before meals, will be sufficient to provide an optimal therapeutical or preventive response. The treatment can be protracted until remission of symptoms, usually for 5-10 days. More prolonged treatments are not contraindicated, considering the poor, if any, toxicity of the components of the formulations of the invention.

The favourable therapeutical results obtained by use of the formulations according to the invention are due to both the synergic interactions between hyaluronic acid, glycyrrhetinic acid and polyvinylpyrrolidone and the capability of the formulation of adhering to the oral mucosa providing a protective coating for the exposed nerve endings, and therefore reduction of pain and promoting cicatrisation and healing of the lesions. Furthermore, the moisturising effect of the compositions has beneficial effect as it protects mucous membranes from further irritating lesions.

The following examples illustrate the invention in greater detail.

### Example 1

### Quali-quantitative composition percent composition

| | |
|---|---|
| Sodium hyaluronate | 0.1 |
| Glycyrrhetinic acid | 0.06 |
| PVP | 9.0 |
| Maltodextrin | 6.00 |
| Propylene glycol | 2.94 |
| Potassium sorbate | 0.3 |
| Sodium benzoate | 0.3 |
| Hydroxyethyl cellulose | 1.5 |
| Hydrogenated castor oil PEG-40 | 0.27 |
| Disodium EDTA | 0.1 |
| Benzalkonium chloride | 0.5 |
| Perfume (Glycyrrhiza Comp. 2717) | 0.16 |
| Sodium saccharin | 0.1 |
| Depurated water | 78.44 |

For the preparation, water is placed in a turboemulsifier, then a mixture of potassium sorbate, sodium benzoate and disodium EDTA is added, followed by hyaluronic acid and maltodextrin. The mixture is stirred after each addition until complete dissolution of the components. After that, PVP is slowly added under stirring and vacuum (30 mm Hg) until complete solvation. Then sodium saccharin and hydroxyethylcellulose are subsequently added, the whole is subjected to vacuum and left under stirring until complete solvation. Afterwards, hydrogenated caster oil 40/OE and perfume, benzalkonium chloride, and a mixture of propylene glycol and glycyrrhetinic acid are added in this order, stirring after each addition until complete dissolution of the components. When the additions are completed, the mixture is stirred under vacuum for 30 minutes.

For the concentrated version of the invention, 10 ml or 15 ml of the composition reported above are distributed in sachets or mono-dose vials, to be diluted with 30-50 ml of water before use; for the ready-to-use version of the invention, the composition reported above is diluted with depurated water to a concentration of 50% and 200 ml or 300ml are distributed in bottles.

### Example 2

### Clinical trials

Thirty patients, of age range from 30 to 60 years, were evaluated, 10 of them being AIDS patients, of age of 30 to 40 years, receiving anti-retroviral therapy. Patients were affected with inflammatory pathologies of the oral cavity of various ætiology:
12 cases of oro-pharyngeal mucositis;
4 cases of aphthous lesions of the oral cavity;
4 cases of post-traumatic lesions;
3 cases of Lichen Planus of the oral cavity;
3 cases of radiotherapy-induced stomatitis;
3 cases of oral cavity surgery side effects;
1 case of leukoplakia.

Patients were treated with the composition of Example 1 in 15 ml sachets diluted in water in a 1:4 ratio. The slightly viscous solution was retained in the mouth for 2-3 minutes during which it was gargled and swirled about to obtain homogeneous distribution on the whole surface of the oral mucosa. The solution was then discharged.

The formulation was used three times a day 60 minutes before meal times for seven consecutive days.

At the end of the treatment, the extent of inflammation and lesions, the decrease or disappearance of dysphagia for solid and semi-solid foods, and liquids, and the duration of the activity of the product were evaluated.

Already after the first administration, more than 80% of patients perceived within a few hours reduction of pain so as to permit food intake. The effect lasted three or four hours.

Healing of the lesions of the oral mucosa already occurred after 3-4 days of treatment in about 60% of treated cases. The percentage reached 90% at the end of the treatment. In the remaining three cases only a pathological condition persisted, but with improved symptoms compared with the beginning of the treatment, providing anyway a remarkable improvement of life quality and restoring a normal, differentiated diet.

### Example 3

Two patients with throat pain (sore throat) were unable to obtain relief with analgesics or other topical agents. Patients were treated with the composition of Example 1 in 15 ml sachets diluted in water in a 1:4 ratio. The solution was retained in the mouth for about one minute during which time it was gargled to obtain good contact with the tissues of the throat. The solution was then discharged. Within ten minutes the patients experienced dramatic relief of their sore throat symptoms, which relief persisted for several hours.

## Claims

1. Pharmaceutical compositions comprising as active ingredients effective doses ranging from 0.01 to 5% by weight of hyaluronic acid, from 0.01 to 3% by weight glycyrrhetinic acid and from 1 to 20% by weight polyvinylpyrrolidone, in mixture with excipients and adjuvants suitable for topical administration.

2. Compositions as claimed in to claim 1, further comprising viscosity-increasing agents, surfactants, stabilising agents-preservatives, flavours, fragrances, sweetening agents, bioadhesive agents, co-solubilisers.

3. Compositions as claimed in claim 2 further comprising cellulose derivatives, acrylic or methacrylic acids polymers or copolymers, ethylene or propylene glycols, polyethoxylated hydrogenated castor oil, EDTA, sodium benzoate, sodium or potassium sorbate, dextrins, sodium saccharin, aspartame.

4. Compositions as claimed in any one of claims 1 to 3 further comprising other active ingredients with complementary or anyway useful activities.

5. Compositions as claimed in claim 4, comprising antibacterials/disinfectants, antifungals, analgesics, anti-inflammatories, emollients, local anæsthetics.

6. A composition according to claim 1 having the following percent composition:
| | |
|---|---|
| Sodium hyaluronate | 0.1 |
| Glycyrrhetinic acid | 0.06 |
| PVP | 9.0 |
| Maltodextrin | 6.00 |
| Propylene glycol | 2.94 |
| Potassium sorbate | 0.3 |
| Sodium benzoate | 0.3 |
| Hydroxyethyl cellulose | 1.5 |
| Hydrogenated castor oil PEG-40 | 0.27 |
| Disodium EDTA | 0.1 |
| Benzalkonium chloride | 0.5 |
| Perfume (Glycyrrhiza Comp. 2717) | 0.16 |
| Sodium saccharin | 0.1 |
| Depurated water | 78.44 |

7. The use of hyaluronic acid, glycyrrhetinic acid and polyvinylpyrrolidone for the preparation of medicaments for the topical treatment of inflammatory conditions of the oral mucosa and lining of the oropharynx and oesophagus, particularly mucositis and stomatitis, and the mucosa of the vagina and rectum, vestibulitis and Behcet's syndrome.

## Patentansprüche

1. Pharmazeutische Zusammensetzungen, umfassend als aktive Bestandteile wirksame Dosen im Bereich von 0,01 bis 5 Gew.-% Hyaluronsäure, 0,01 bis 3 Gew.-% Glycyrrhetinsäure und 1 bis 20 Gew.-% Polyvinylpyrrolidon im Gemisch mit Exzipienten und Hilfsstoffen, die für die topische Verabreichung geeignet sind.

2. Zusammensetzungen wie in Anspruch 1 beansprucht, darüber hinaus umfassend viskositätserhöhende Mittel, oberflächenaktive Stoffe, stabilisierende Mittel-Konservierungsmittel, Geschmacksstoffe, Duftstoffe, Süßungsmittel, bioadhäsive Mittel, Co-Lösungsvermittler.

3. Zusammensetzungen wie in Anspruch 2 beansprucht, darüber hinaus umfassend Cellulose-Derivate, Acryl- oder Methacrylsäurepolymere oder -copolymere, Ethyl- oder Propylenglykole, polyethoxyliertes hydriertes Castoröl, EDTA, Natriumbenzoat, Natriumoder Kaliumsorbat, Dextrine, Saccharin-Natrium, Aspartam.

4. Zusammensetzungen wie in einem der Ansprüche 1 bis 3 beansprucht, darüber hinaus umfassend weitere aktive Bestandteile mit komplementären oder jedenfalls nützlichen Aktivitäten.

5. Zusammensetzungen wie in Anspruch 4 beansprucht, umfassend antibakterielle Mittel/Desinfektionsmittel, Antipilzmittel, Analgetika, antientzündliche Mittel, erweichende Mittel, Lokalonästhetika.

6. Zusammensetzung nach Anspruch 1, welche die folgende prozentuale Zusammensetzung aufweist:
| | |
|---|---|
| Natriumhyaluronat | 0,1 |
| Glycyrrhetinsäure | 0,06 |
| PVP | 9,0 |
| Maltodextrin | 6,00 |
| Propylenglykol | 2,94 |
| Kaliumsorbat | 0,3 |
| Natriumbenzoat | 0,3 |
| Hydroxyethylcellulose | 1,5 |
| Hydriertes Castoröl PEG-40 | 0,27 |
| Dinatrium-EDTA | 0,1 |
| Benzalkoniumchlorid | 0,5 |
| Parfüm (Glycyrrhiza Comp. 2717) | 0,16 |
| Saccharin-Natrium | 0,1 |
| Gereinigtes Wasser | 78,44 |

7. Verwendung von Hyaluronsäure, Glycyrrhetinsäure und Polyvinylpyrrolidon zur Herstellung eines Medikaments zur topischen Behandlung von entzündlichen Zuständen der Mundschleimhaut und Oropharynx- und Oesophagus-Wand, insbesondere Mucositis und Stomatitis, und der Schleimhaut von Vagina und Rektum, Vestibulitis und Behcet-Syndrom.

## Revendications

1. Compositions pharmaceutiques comprenant, en tant qu'ingrédients actifs, des doses se situant dans les plages de 0,01 à 5% en poids d'acide hyaluronique, de 0,01 à 3% en poids d'acide glycyrrhétinique et de 1 à 20% en poids de polyvinylpyrrolidone, en mélange avec des excipients et des adjuvants appropriés pour une administration par voie topique.

2. Compositions telles que revendiquées dans la revendication 1, comprenant en outre des agents d'augmentation de la viscosité, des surfactants, des agents-conservateurs stabilisants, des agents arômatisants, des parfums, des agents sucrants, des agents bioadhésifs, des agents de co-solubilisation.

3. Compositions telles que revendiquées dans la revendication 2, comprenant en outre des dérivés cellulosiques, des -polymères ou copolymères d'acide acrylique ou méthacrylique, des éthylène ou propylène glycols, de l'huile de ricin hydrogénée polyéthoxylée, de l'EDTA, du benzoate de sodium, du sorbate de sodium ou de potassium, des dextrines, de la saccharine sodique, de l'aspartame.

4. Compositions telles que revendiquées dans l'une quelconque des revendications 1 à 3, comprenant en outre d'autres ingrédients actifs avec des activités complémentaires ou de toute façon utiles.

5. Compositions telles que revendiquées dans la revendication 4, comprenant des antibactériens/désinfectants, des antifongiques, des analgésiques, des anti-inflammatoires, des émollients, des anesthésiques locaux.

6. Composition selon la revendication 1, ayant la composition suivante en pourcent :
| | |
|---|---|
| Hyaluronate de sodium | 0,1 |
| Acide glycyrrhétinique | 0,06 |
| PVP | 9,0 |
| Maltodextrine | 6,00 |
| Propylène glycol | 2,94 |
| Sorbate de potassium | 0,3 |
| Benzoate de sodium | 0,3 |
| Hydroxyéthylcellulose | 1,5 |
| Huile de ricin hydrogénée PEG-40 | 0,27 |
| EDTA disodique | 0,1 |
| Chlorure de benzalkonium | 0,5 |
| Parfum (Glycyrrhiza Comp. 2717) | 0,16 |
| Saccharine sodique | 0,1 |
| Eau épurée | 78,44 |

7. Utilisation de l'acide hyaluronique, de l'acide glycyrrhétinique et de la polyvinylpyrrolidone pour la préparation de médicaments pour le traitement par voie topique d'états inflammatoires de la muqueuse orale et de la paroi de l'oropharynx et de l'oesophage, en particulier de la mucosité et de la stomatite, de la muqueuse du vagin et du rectum, de la vestibulite et du syndrome de Behcet.
